Europäisches Patentamt

European Patent Office

Office européen des brevets

(11) Numéro de publication: **0 139 592 B1**

# FASCICULE DE BREVET EUROPEEN

(45) Date de publication de fascicule du brevet: **25.03.92** (51) Int. Cl.⁵: **C12M 1/12**

(21) Numéro de dépôt: **84402091.7**

(22) Date de dépôt: **17.10.84**

(54) **Procédé et installation de production d'amino-acides par fermentation.**

(30) Priorité: **27.10.83 FR 8317194**

(43) Date de publication de la demande:
**02.05.85 Bulletin 85/18**

(45) Mention de la délivrance du brevet:
**25.03.92 Bulletin 92/13**

(84) Etats contractants désignés:
**AT BE CH DE FR GB IT LI LU NL SE**

(56) Documents cités:
**EP-A- 0 065 895**
**FR-A- 1 590 050**
**FR-A- 2 430 451**
**US-A- 3 399 114**
**US-A- 3 647 633**

**CHEMICAL ABSTRACTS, vol. 88, no. 11, 13
mars 1978, page 386, résumé no. 73014j,
Columbus, Ohio, US; & JP-A-77 136 985
(AJINOMOTO CO. INC.) 16-11-1977**

**MICROBIOLOGY ABSTRACTS, vol. 14, no. 7,
juillet 1979, page 2, résumé no. 4916-A-14,
Londres, GB; S.P. CHATTOPADHYAY et al.:
"Production of valine by a Bacillus sp.", & Z.
ALLG. MIKROBIOL. 18(4), 243-254 (1978)**

**CHEMICAL ABSTRACTS, vol. 104, no. 1, janvier 1986, page 427, résumé no. 4641y, Columbus, Ohio, US; & JP-A-60 168 394 (TORAY
INDUSTRIES INC.) 31-08-1985**

(73) Titulaire: **LES PRODUITS ORGANIOUES DU
SANTERRE ORSAN
16 rue Ballu
F-75009 Paris(FR)**

(72) Inventeur: **Renaud, Claude Marcel
6 Faubourg St. Léonard
F-80190 Nesle(FR)**
Inventeur: **Pons, Benoit Joseph
rue des Marroniers Longuevoisin
F-80190 Nesle(FR)**
Inventeur: **Petroff, Dominique
49 avenue Mac Orlawd
F-80200 Peronne(FR)**

(74) Mandataire: **Portal, Gérard et al
Cabinet Z. Weinstein 20, Avenue de Friedland
F-75008 Paris(FR)**

## Description

La présente invention concerne essentiellement un procédé et une installation de production d'amino-acides par fermentation.

On connaît déjà, notamment par les brevet Asahi délivrés aux USA sous les N°ˢ USP 3.355 359 & 3.399 144, un procédé de production d'acide glutamique par fermentation comprenant, dans une zone de fermentation et dans des conditions de fermentation appropriées, la fermentation d'une souche productrice d'acide glutamique sur un milieu de fermentation approprié, de préférence avec un ajout d'un substrat nutritif au cours de la fermentation, ce procédé étant dénommé procédé "fed-batch". Selon ce procédé, on débute la fermentation avec un faible niveau de milieu de fermentation dans le réacteur, ce niveau augmentant régulièrement lors de l'ajout, habituellement continu, de substrat nutritif au cours de la fermentation, pour aboutir à un niveau supérieur dans le réacteur pour lequel on arrête la fermentation.

La fermentation est en général réalisée avec une souche productrice d'acide glutamique constituée par une souche de Corynebacterium melassecola qui exige la présence de biotine comme facteur de croissance. Selon le niveau de biotine, il est nécessaire d'introduire un ou plusieurs additifs appropriés. Si le milieu est peu riche en biotine, on ajoute un seul additif et dans le cas où le milieu est riche en biotine, on ajoute au minimum deux additifs.

Ces procédés connusdu type "fed-batch" présentent l'inconvénient majeur que, lors de l'ajout successif de substrat nutritif en cours de fermentation, une proportion importante de matières diverses, organiques et minérales, s'accumulent dans le fermenteur au fur et à mesure que la fermentation avance en âge et finissent par atteindre une concentration critique formant en quelque sorte un seuil de toxicité pour la phase de production de l'acide glutamique ce qui limite par conséquent le rendement et la productivité en acide glutamique.

Il en résulte que les procédés de type "fed-batch" sont limités en rendement et en productivité ce qui en constitue un grave inconvénient pour une production industrielle.

Ainsi, compte tenu de l'état de la technique précédent, l'invention a pour but de résoudre le problème technique consistant en la production d'acide glutamique par fermentation, au niveau industriel avec un rendement et une productivité radicalement accrus par rapport aux procédés antérieurs et en particulier du type "fed-batch".

D'autre part, l'invention a également pour but de résoudre le problème technique de l'obtention d'une concentration d'acide glutamique telle que les opérations futures d'extraction de celui-ci soient réalisables dans de bonnes conditions techniques et économiques.

Ces divers problèmes techniques sont résolus pour la première fois par la présente invention.

En outre, l'invention a généralement pour but de produire d'autres amino-acides par fermentation avec les même avantages techniques.

Ainsi, la présente invention,selon l'un de ses aspects, fournit un procédé de production d'amino-acide par fermentation comprenant, dans une zone de fermentation et dans des conditions de fermentation appropriés, la fermentation d'une souche productrice dudit amino-acide sur un milieu de fermentation approprié, de préférence avec ajout d'un substrat nutritif en cours de fermentation selon le procédé "fed-batch", caractérisé en ce que, lorsque l'on atteint une concentration prédéterminée en amino-acide, on procède à une filtration appropriée du milieu de fermentation en obtenant d'une part un rétentat contenant l'amino-acide ; on recycle au moins en partie le rétentat dans la zone de fermentation et on recueille le filtrat pour de préférence en séparer ensuite l'amino-acide ; et, lors de cette étape de filtration, on bloque sensiblement la croissance de la souche.

Ce blocage de la croissance de la souche varie naturellement en fonction de la souche productrice d'amino-acide utilisée pour produire l'amino-acide en question. L'homme du métier de la fermentation connaît parfaitement le moyen de bloquer la croissance de chaque souche particulière. Pour certaines souches, comme en particulier celles qui produisent l'acide glutamique, on réalise ce blocage de la croissance de la souche, lors de l'étape de filtration, par ajout par intermittence ou en continu dans la zone de fermentation d'au moins un agent bloquant sensiblement la croissance de la souche. De préférence, cet agent bloquant la croissance de la souche est un dérivé d'acide gras, de préférence choisi parmi un ester d'acide gras de polyoxyéthylène, avantageusement l'ester monopalmitate ou monostéarate de polyoxyéthylène, ou encore un sel d'acylamine, avantageusement l'acétate de laurylamine ou de myristylamine.

Pour d'autres amino-acides, comme en particulier dans le cas de la valine, on réalise ce blocage de la croissance de la souche en suppliment simplement dans le substrat nutritif une substance essentielle à la croissance de la souche, substance qui est normalement ajoutée lors de la fermentation. Par exemple, dans le cas de la valine, on supprime la présence d'isoleucine qui était auparavant présente dans le milieu de fermentation, de sorte que lorsque la souche productrice de valine a épuisé l'isoleucine présente dans le milieu, sa croissance est sensiblement ou essentiellement bloquée.

Selon l'invention, on ne peut naturellement produire que les amino-acides (ou acides aminés) qui peuvent être produits par fermentation. Ces amino-acides sont bien connus à l'homme du métier ainsi que les souches productrices de tels amino-acides par fermentation. Des amino-acides qui sont de préférence produits selon l'invention sont l'acide glutamique, la valine, l'acide aspartique, l'arginine, l'histidine, l'isoleucine, la leucine, la méthionine, l'ornithine, la phénylalanine, la proline et la thréonine. On préfère particulièrement produire l'acide glutamique et la valine car les performances sont particulièrement exceptionnelles.

Lorsque la fermentation est aérobie, ce qui est habituellement le cas, le procédé selon l'invention est de préférence caractérisé en ce qu'on réalise l'aération en faisant passer le rétentat recyclé dans un hydro-injecteur avant son recyclage dans la zone de fermentation.

De préférence, on procède à la filtration lorsque la concentration en amino-acide arrive à un palier, c'est-à-dire par exemple dans le cas de l'acide glutamique, lorsque la concentration est égale ou supérieure à environ 60 grammes par litre de milieu de fermentation.

Selon une autre caractéristique du procédé selon l'invention, on réalise la filtration en continu et on introduit du substrat nutritif dans la zone de fermentation en quantité suffisante pour compenser la quantité de substrat soutirée de la zone de fermentation et non réintroduite.

Ainsi, selon l'invention, on maintient la concentration en amino-acide à une valeur sensiblement constante et également une biomasse maintenue sensiblement constante ce qui aboutit, de manière totalement inattendue, à une augmentation radicale du rendement et de la productivité en amino-acide.

Le rendement en amino-acide est exprimé en masse d'amino-acide rapportée à la masse de sucre mis en oeuvre pour la fermentation tandis que la productivité en amino-acide est exprimée en masse d'amino-acide par litre de fermenteur utile et par heure de fermentation.

Ces résultats seront mis en évidence dans les exemples comparatifs donnés plus loin.

On a pu démontrer à ce sujet que ces résultats tout à fait inattendus sont dus à la combinaison de la filtration et de l'étape de blocage de la croissance de la souche.

En effet, par exemple la simple combinaison de la filtration avec le procédé connu de production d'acide glutamique par fermentation, par exemple le procédé type "fed batch" aboutit à une diminution radicale de la productivité de la souche et ceci sera démontré plus loin par l'exemple N° 6 de comparaison.

Ainsi, bien que la technique de recyclage des cellules par filtration ait déjà été utilisée dans les procédés de fermentation (voir par exemple la revue "Process Biochemistry, Août/Septembre 1980, pages 7 à 10 ; ou la demande brevet européen N° 82 400 775.1 publiée sous le N° 65 895, page 2, ligne 6 à page 5 ligne 25 et les revendications), ce recyclage des cellules par filtration conduit à une augmentation importante de la biomasse ce qui était considéré jusqu'alors comme un facteur déterminant pour l'augmentation du rendement et de la productivité.

L'invention, de manière inattendue, au contraire, limite volontairement la croissance de la biomasse (donc de la souche) lors de l'étape de filtration,(notamment par la suppression d'une substance nécessaire à la croissance ou l'ajout par intermittence ou en continu d'agent(s) bloquant, la croissance de la souche)ce qui aboutit d'une manière inattendue à un accroissement radical du rendement et de la productivité de la souche. D'autre part, la concentration d'amino-acide est telle que l'extraction de l'amino-acide est réalisable dans de bonnes conditions techniques et économiques.

L'invention concerne également selon un autre de ses aspects, une installation de production d'amino-acide par fermentation, comprenant un fermenteur, des moyens d'alimentation en milieu de fermentation ou nutritif audit fermenteur, des moyens de soutirage de milieu fermenté du fermenteur ; et de préférence des moyens d'agitation ainsi que des moyens d'aération du milieu de fermentation, caractérisée en ce que les moyens de soutirage sont reliés à l'entrée d'une unité de filtration comprenant une sortie de rétentat et une sortie de filtrat, de préférence la sortie de rétentat est reliée audit fermenteur pour réaliser au moins en partie un recyclage du rétentat ; et des moyens d'introduction dans le fermenteur d'au moins un agent bloquant la croissance de la souche.

Selon un mode de réalisation de cette installation, l'unité de filtration comprend au moins un module de filtration tangentielle. Selon un autre mode de réalisation de cette installation, l'unité de filtration comprend au moins un module de filtration, de préférence tubulaire, à membrane de préférence minérale.

Selon un mode de réalisation particulièrement avantageux de cette installation, les moyens d'agitation et/ou d'aération comprennent un hydro-injecteur sur le circuit de recyclage du rétentat au fermenteur dans lequel passe le rétentat recyclé au fermenteur.

Les amino-acides qui peuvent être produits et les souches capables de produire des amino-acides sont naturellement tels que précédemment définis pour le procédé.

D'autres buts, caractéristiques et avantages de l'invention apparaîtront clairement à la lumière de la description explicative qui va suivre relative à plusieurs exemples de l'art antérieur et comprenant plusieurs exemples de réalisation selon l'invention, en référence également aux dessins annexés dans lesquels :

La figure 1 représente schématiquement une installation de base de production d'amino-acide montrant principalement le fermenteur ;

La figure 2 représente le couplage du fermenteur avec une cellule de filtration selon l'invention ;

La figure 3 représente un mode de réalisation d'une cellule de filtration du type tangentielle ;

La figure 4 représente un autre mode de réalisation de module de filtration de type tubulaire à membrane minérale ;

La figure 5 représente un mode de réalisation préféré de l'invention selon l'invention comprenant un fermenteur, une cellule de filtration et un hydro-injecteur ; et

La figure 6 représente une vue agrandie de détail de l'hydro-injecteur représenté à la figure 5.

Les exemples et les modes de réalisation ne sont donnés naturellement qu'à titre d'illustration et ne sauraient donc en aucune façon limiter la portée de l'invention.

## I. EXEMPLES RELATIFS A LA PRODUCTION D'ACIDE GLUTAMIQUE Exemple N° 1 de comparaison

Cet exemple est relatif au procédé connu par le brevet Asahi USP N° 3.355 359 de fermentation glutamique de type "fed-batch"en présence d'une concentration en biotine (facteur de croissance)- insuffisante.

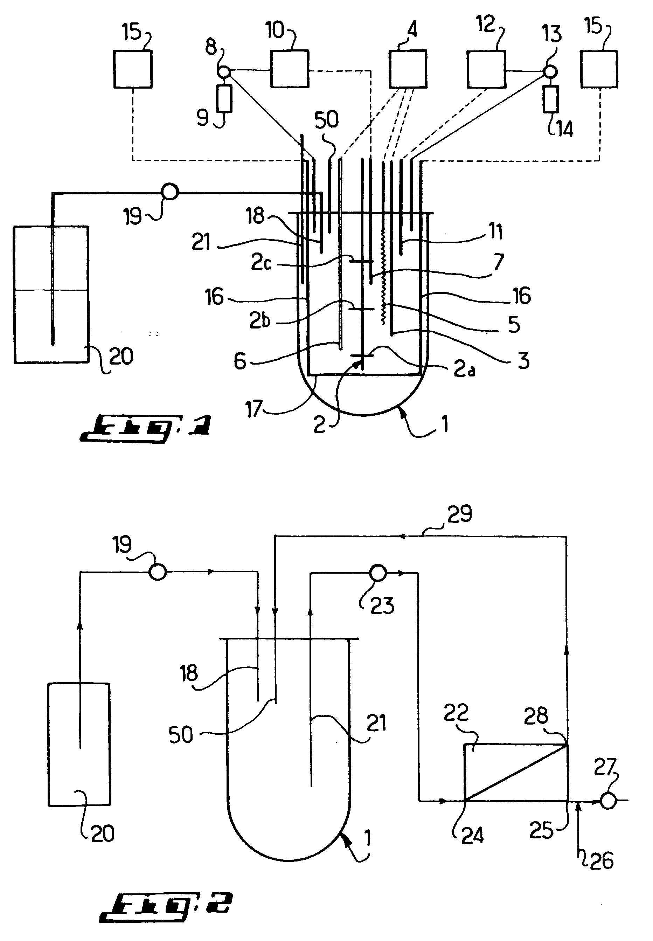

Pour réaliser cet exemple, on utilise l'installation simplifiée de la figure 1 et qui comprend principalement un fermenteur 1 d'une capacité totale de 10 litres, et de volume utile de 7 litres. Ce fermenteur est pourvu de moyens d'agitation 2 par exemple formés par un axe à trois turbines 2a, 2b, 2c ;

Une sonde 3 et un régulateur de température 4 actionnent des moyens de chauffage 5 par résistance et le refroidissement du réacteur par circulation d'eau dans une baffle creuse 6.

On prévoit également une sonde de contact 7 commandant une pompe 8 d'introduction d'un produit anti-mousse stocké dans un réservoir approprié 9 par l'intermédiaire d'un relais 10.

On prévoit également une sonde de pH 11 reliée à un bloc de mesure et régulation 12 commandant la pompe 13 d'une solution d'ammoniaque 14.

Le fermenteur est également pourvu de moyens d'aération formés ici par une réserve d'air stérile 15 reliée à des tubes plongeants 16 et un diffuseur annulaire 17.

Le fermenteur est également pourvu de moyens d'alimentation 18 en substrat nutritif pompé par une pompe 19 à partir d'une réserve 20 en substrat nutritif.

On prévoit également des moyens 21 de soutirage de milieu fermenté, formés ici simplement par un tube plongeant de prélèvement.

Le fermenteur est également pourvu de moyens 50 formés par exemple par des tubes plongeants permettant l'introduction de divers produits lors de la fermentation tels que inoculum, $NH_3$, mélasse , rétentat, etc.

Dans cet exemple de comparaison selon le procédé Asahi précité, on introduit dans le fermenteur un milieu de fermentation (A) dont la composition est donnée dans le tableau I en fin de description et le pH est ajusté et régulé à 7,3 avec une solution d'ammoniaque.

Ce milieu est ensemencé à 50 % (volume/volume) par une culture de Corynebacterium melassecola, cultivée sur le milieu de culture B donné également dans le tableau I et l'agitation est mise en place.

La température de fermentation est réglée à 35° et le débit d'air est de 0,8 V/V. minute.

La concentration initiale en biotine (facteur de croissance) est de 5 microgrammes par litre. A cette faible concentration il est seulement nécessaire l'emploi d'un seul tensio-actif constitué par du polyoxyéthylène monopalmitate à une concentration de 2 grammes par litre.

Le milieu est alimenté avec un substrat nutritif depuis la réserve 20 du type mélasse de betterave dont la concentration en sucre est de 500 grammes par litre. Cette alimentation est réalisée selon un débit suffisant pour maintenir une concentration résiduelle en sucre de 30 grammes par litre. La durée totale de la fermentation est de 34 heures.

La concentration finale en acide glutamique est de 90 grammes par litre avec un rendement d'acide glutamique rapporté au sucre mis en oeuvre de 55 % et une productivité en acide glutamique par litre de fermenteur utile et par heure de 2,7 (c'est-à-dire 2,7 grammes/litre.heure).

## Exemple N° 2 de comparaison

Cet exemple est un exemple de comparaison de fermentation glutamique de type "fed-batch" en présence d'un excès de biotine comme facteur de croissance, ce qui exige l'addition de deux tensio-actifs.

Ce procédé est réalisé selon le brevet Asahi USP N° 3.399 114.

Selon cet exemple on utilise également le milieu de fermentation A du tableau I mais dans lequel la concentration initiale en biotine est de 100 microgrammes par litre. La procédure est identique à l'exemple 1 avec également alimentation en milieu nutritif comme décrit à l'exemple 1.

L'addition du premier tensio-actif (polyoxyéthylène monopalmitate) est réalisée lorsque la densité optique nette est égale à 0,35 avec une concentration de 1 gramme par litre ; et 1 heure après l'addition du premier tensio-actif, quand la densité optique nette est de 0,65, on ajoute le deuxième tensio-actif (acétate de laurylamine) à une concentration de 0,3 gramme par litre.

On arrête la fermentation après 16 heures de fonctionnement. La concentration finale en acide glutamique est de 65 grammes par litre avec un rendement de 49 % et une productivité de 4 grammes/litre.heure.

Exemple N° 3 de comparaison

Cet exemple de comparaison est relatif à une fermentation glutamique de type "fed batch" toujours en présence d'un excès de biotine et emploi de deux agents tensio-actifs nécessité par l'excès de biotine.

On procède comme décrit à l'exemple 2 mais la durée totale de fermentation est de 34 heures au lieu de 16 heures.

La concentration finale en acide glutamique est de 100 grammes par litre soit un rendement de 58,5 % et une productivité de 3 grammes/litre.heure.

Exemple N° 4 selon l'invention

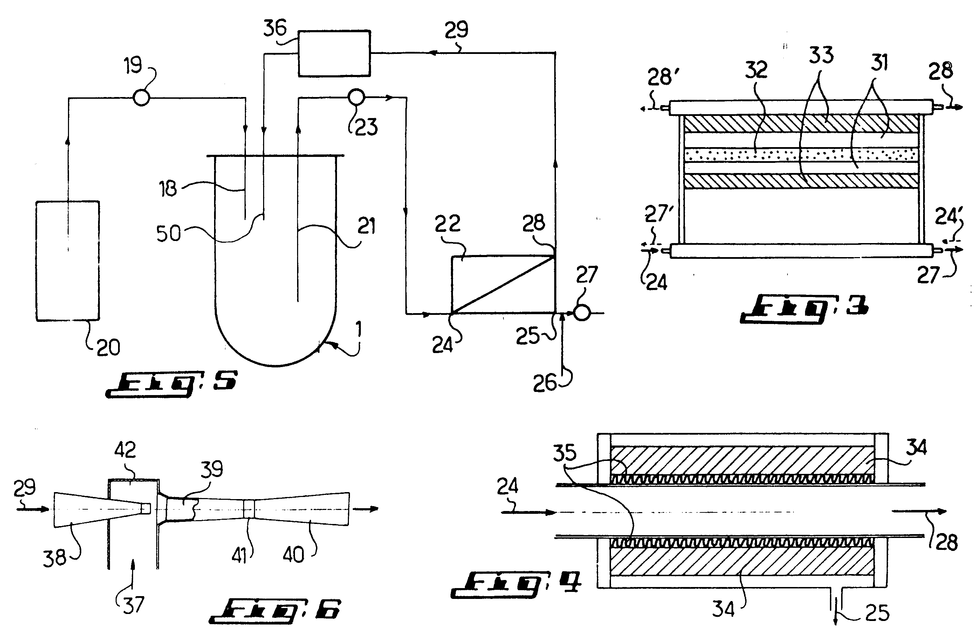

Selon l'invention, on utilise l'installation représentée à la figure 2 couplant un module de filtration avec le fermenteur.

Selon cet exemple, la cellule de filtration est la cellule de filtration tangentielle représentée à la figure 3 et qui présente une entrée 24 reliée par une pompe 23 aux moyens de soutirage 21 et deux sorties :

une sortie filtrat 25 reliée par une conduite 26 à une pompe 27 et une sortie rétentat 28 permettant le recyclage du rétentat dans le fermenteur 1 par la conduite 29 et le tube plongeant 50.

La cellule de filtration tangentielle est constituée par deux paquets 30 d'une série de filtres représentant une surface totale de filtration de 900 cm$^2$. Chaque paquet comprend deux membranes 31 dont le diamètre de pores est de 0,45 micron, séparées par un séparateur de filtrat 32. Un séparateur de rétentat 33 est intercalé entre les deux paquets. L'ensemble est stérilisé chimiquement à l'eau de javel. Ce type de cellule de filtration tangentielle est couramment disponible sur le marché.

Selon le procédé de l'invention, la première étape du procédé de fermentation est réalisée de préférence selon le procédé "fed-batch" et est donc, pour une concentration en biotine insuffisante, réalisée comme décrit à l'exemple 1, c'est-à-dire tout d'abord sans l'utilisation de la cellule de filtration.

Cependant, lorsque l'on arrive à une concentration prédéterminée en acide glutamique, qui est de préférence égale ou supérieure à 60 grammes par litre, c'est-à-dire dans le cas présent après 16 heures de fermentation, on met en fonctionnement la cellule de filtration 22 par actionnement de la pompe 23 selon un taux de recyclage de 6 litres par heure.

D'autre part, l'alimentation est réalisée avec un milieu nutritif de plus faible concentration en glucose, c'est-à-dire dans le cas présent avec une mélasse dont la concentration en sucre est de 100 grammes par litre et le débit de soutirage et d'alimentation est identique pour maintenir le niveau constant dans le fermenteur. Ce débit est sélectionné pour maintenir un taux de dilution de 0,06 h$^{-1}$.

Au cours de la deuxième étape de filtration, on ajoute toutes les deux heures un agent bloquant la croissance de la souche productrice d'acide glutamique qui est de préférence un dérivé d'acide gras. L'agent tensio-actif constitué par le polyoxyéthylène mono-palmitate qui est un ester d'acide gras a été trouvé satisfaisant et celui-ci est ajouté toutes les 2 heures à une concentration de 1 gramme par litre.

La durée de la fermentation totale est de 34 heures, soit 18 heures de fermentation avec la filtration des cellules de la souche.

On observe que la concentration en acide glutamique déterminée dans le filtrat pompé par la pompe 27 de la conduite 26 se stabilise à la valeur de 60 grammes par litre et on obtient un rendement de 53 % et une productivité de 3,6 grammes par litre. heure.

On constate ainsi que la productivité est accrue de manière tout à fait inattendue par rapport à l'exemple de comparaison de l'exemple N° 1.

Exemple N° 5 selon l'invention

Dans cet exemple, on réalise une fermentation glutamique avec un milieu de fermentation ayant un excés de biotine, soit à une concentration initiale en biotine de 100 microgrammes par litre comme à l'exemple N° 2.

Ainsi, on réalise tout d'abord la fermentation comme décrit dans l'exemple N° 2 où on aboutit après 16 heures de fonctionnement à une concentration en acide glutamique de 65 grammes par litre.

L'installation utilisée est naturellement celle de la figure 2 avec le module de filtration tangentielle de la figure 3.

On déclenche alors la filtration avec un taux de recyclage de 6 litres par heure et un taux de dilution maintenu à 0,06 $h^{-1}$. On utilise également un milieu nutritif à teneur réduite en sucre, en l'occurrence une mélasse de betterave avec une concentration en sucre de 100 grammes par litre.

On ajoute périodiquement et alternativement deux agents bloquant la croissance de la souche, le premier étant un ester d'acide gras constitué de préférence par le polyoxyéthylène monopalmitate et le second étant un ester d'acylamine de préférence constitué par l'acétate de laurylamine. Cette addition périodique et alternative de ces deux agents bloquants est réalisée toutes les heures avec des concentrations respectives de 0,5 gramme par litre et 0,15 gramme par litre.

La durée totale de la fermentation est de 34 heures. On observe que la concentration en acide glutamique se stabilise à la valeur de 65 grammes par litre avec un rendement de 54,5 % et une productivité de 3,9 grammes/litre.heure.

Cette productivité est à rapprocher de celle obtenue à l'exemple 3 pour une même durée de fermentation. On observe encore un gain radical en productivité, de pratiquement un tiers soit environ 30 % de gain de productivité.

Exemple N° 6 de comparaison

Cet exemple a pour but de montrer l'effet critique de la combinaison de la filtration et de l'addition d'au moins un agent bloquant la croissance de la souche.

Pour ce faire, on procède comme décrit à l'exemple N° 5 sauf qu'on ne réalise aucune addition d'agent bloquant la croissance de la souche, c'es-à-dire que l'on n'additionne pas de tensio-actif pendant la phase de filtration.

La durée totale de la fermentation est également de 34 heures et on observe que la concentration en acide glutamique diminue progressivement de 65 à 20 grammes par litre avec une productivité moyenne de 1,7 gramme/litre.heure.

On observe ainsi que la simple combinaison de la filtration avec la fermentation aboutit à une baisse radicale de la productivité qui est presque de 50 %.

Cette baisse de productivité apparaît être due à une croissance incontrôlée de la souche qui arrête la production d'acide glutamique.

Exemple N° 7 selon l'invention

On procède ici comme à l'exemple N° 5 sauf que l'addition des deux agents bloquant la croissance de la souche est effectuée en continu à raison de 0,25 gramme/litre.heure et 0,08 gramme/litre.heure. respectivement.

La durée totale de la fermentation est également de 34 heures. La concentration en acide glutamique s'est stabilisée à la valeur moyenne de 70 grammes/litre, ce qui représente un rendement de 56 % et une productivité de 4,1 grammes/litre.heure.

Exemple N° 8 selon l'invention

On procède comme décrit à l'exemple N° 7 mais la durée totale de fermentation est portée à 70 heures au lieu de 34 heures. La concentration en acide glutamique s'est stabilisée à la valeur moyenne de 69 grammes/litre, soit un rendement de 61 % et une productivité de 4 grammes/litre.heure.

Cet exemple permet de constater que même en doublant la durée de fermentation la productivité de la souche est maintenue à un niveau nettement supérieur à celui atteint selon l'art antérieur, et ce d'une manière tout à fait inattendue.

Exemple N° 9 selon l'invention

Dans une première étape, on utilise le procédé de fermentation "fed-batch" décrit dans l'exemple N° 2 à la suite duquel on déclenche la filtration. Le taux de dilution est maintenu à la valeur de 0,1 h$^{-1}$ avec une mélasse d'alimentation dont la concentration en sucre est de 75 grammes par litre et un taux de recyclage de 6 litres par heure.

Lors de la filtration, on ajoute deux agents bloquant la croissance de la souche décrits à l'exemple 7 et selon les mêmes modalités.

La durée totale de la fermentation est de 34 heures comme à l'exemple 7. La concentration en acide glutamique se stabilise à la valeur moyenne de 40 grammes par litre soit un rendement de 51 % et une productivité de 4 grammes/litre.heure.

Il est à noter que comme dans les cas précédents d'utilisation du module de filtration tangentielle représenté à la figure 3, toutes les 6 heures, on inverse le sens de filtration, la sortie 27 devenant ainsi l'entrée 24', l'entrée 24 devenant la sortie 27', la sortie 28 étant remplacée par la sortie 28' comme représenté à la figure 3. Ceci évite tout colmatage des membranes.

Exemple N° 10 selon l'invention

Dans cet exemple, on utilise comme souche une souche de Corynebacterium glutamicum.

On réalise la première étape de fermentation par "fed-batch" comme décrit à l'exemple N° 2. On déclenche ensuite la filtration avec le module de filtration à membrane minérale de la figure 4. Le taux de dilution est maintenu à la valeur de 0,06 h$^{-1}$ avec un milieu nutritif constitué par une mélasse de betterave à 100 grammes/litre et un taux de recyclage de 800 litres/heure.

On ajoute les deux agents de blocage de la croissance de la souche décrits à l'exemple N° 5 selon les mêmes modalités, c'est-à-dire alternativement toutes les deux heures avec respectivement une concentration de 0,5 et 0,15 gramme par litre.

La durée totale de la fermentation est de 34 heures.

La concentration en acide glutamique se stabilise à une valeur moyenne de 67 grammes/litre avec un rendement de 55 % et une productivité de 3,9 grammes/litre. heure.

Exemple N° 11 selon l'invention

Les conditions de fermentation sont identiques à celles décrites dans l'exemple N° 10 mais la durée totale de la fermentation est portée à 70 heures.

La concentration en acide glutamique se stabilise à la valeur moyenne de 68 grammes/litre, soit un rendement de 55,5 % et une productivité de 4 grammes/litre.heure.

Ceci confirme une fois de plus qu'en doublant la durée de la fermentation la productivité est au moins maintenue à la valeur très élevée obtenue selon l'invention .

Exemple N° 12 selon l'invention

Dans cet exemple, les conditions de fermentation sont identiques à celles décrites dans l'exemple 10 sauf qu'au lieu d'effectuer l'agitation par les moyens d'agitation 2 et l'aération par les moyens d'aération 15, on utilise l'installation de la figure 5 comportant la présence d'un hydro-injecteur 36 dans le circuit de recyclage du rétentat par le fermenteur 1.

Le schéma de principe de l'hydro-injecteur 36 est donné à la figure 6. Le fluide issu de la sortie rétentat 28 est amené par la conduite 29 à l'entrée de la tuyère motrice 38 en provoquant une aspiration d'air par le conduit 37 dans la chambre 42. Cette aspiration d'air résulte en la formation d'une émulsion air-fluide à l'intérieur du mélangeur 39 qui est ensuite diffusée au niveau du fermenteur 1 par le système de diffusion 41, 40 relié au moyen d'introduction 50 de rétentat recyclé.

L'hydro-injecteur est réglé de manière à réaliser une aération similaire à celle de l'exemple N° 1. Les conditions de fermentation sont identiques à celles décrites dans l'exemple 10 avec une durée totale de fermentation de 34 heures.

La concentration en acide glutamique s'est stabilisée à la valeur moyenne de 72 grammes/litre soit un rendement de 57 % et une production de 4,2 grammes/litre.heure,ce qui est tout à fait remarquable.

En ce qui concerne le module de filtration tubulaire à membrane minérale de la figure 4, celui-ci comprend un support poreux en graphite 34 et une membrane minérale en oxyde de zirconium 35 présentant un seuil de coupure de 100.000. L'ensemble est stérilisé à la vapeur.

II. EXEMPLES DE REALISATION RELATIVEMENT A LA PRODUCTION DE VALINE

Dans le cas de la production de valine, la souche de micro-organisme productrice de valine utilisée et la souche Corynebacterium glutamicum mutée déposée à l'Institut Pasteur sous le N°l026 le 14 Avril 1976, qui a également été déposée au Fermentation Research Institute, au Japon, sous le N° FERM-4035 le 21 Avril 1977, cette souche étant décrite dans le brevet français antérieur de la demanderesse FR-76 12326.

Cette souche est utilisée dans les trois exemples suivants qui sont réalisés dans le même fermenteur. D'autre part, une unité de filtration (22) comprend de préférence un module de filtration tubulaire à membrane minérale , tel que décrit et représenté à la figure 4.

Le milieu de fermentation et de culture est le milieu C indiqué au tableau II en fin de description tandis que le substrat nutritif est le milieu D indiqué au tableau II en fin de la description.

Les conditions de fermentation sont généralement les suivantes :

La température est régulée à 30°C et le pH est réglé à 8 avec une solution d'ammoniaque ; l'aération à 0,8 VVm ; l'agitation est de 600 tours par minute.

Exemple N° 13 de comparaison

Dans cet exemple, on réalise la production de valine selon le procédé Batch.

On introduit le milieu de fermentation C du tableau II dans le fermenteur et on ensemence par une culture de la souche Corynebacterium glutamicum précitée, à 5 % (valine/volume).

Après 36 heures de fermentation, le sucre est épuisé. La concentration finale en biomasse est de 9,9 grammes/litre et la teneur finale en valine est de 17,1 grammes/litre.

Le rendement en valine, rapporté au suce consommé, est de 19 % et la productivité en valine est de 0,48 gramme/litre.heure.

Exemple N° 14 de comparaison

Dans cet exemple de comparaison, on réalise la production de valine en procédé continu.

On démarre la fermentation selon les conditions opératoires décrites à l'exemple 13, en procédé Batch, d'une durée de 30 heures. A ce temps, on procède à l'alimentation continue du fermenteur (1) avec le milieu nutritif de composition identique au milieu de fermentation C du tableau II. Le débit d'alimentation ainsi que le débit de soutirage sont identiques et imposent un taux de dilution de 0,05 $h^{-1}$.

Un régime stationnaire s'établit et est maintenu pendant 60 heures.

La concentration en biomasse à l'équilibre est de 9 grammes /litre et la concentration en valine est de 14,3 grammes /litre. La durée totale de la fermentation est de 90 heures.

La productivité en valine est de 0,64 gramme /litre.heure et le rendement de valine rapporté au sucre consommé est de 17 %.

Exemple N° 15 selon l'invention

Dans cet exemple, on réalise la production de valine en continu en utilisant l'installation représentée à la figure 2 couplée à un module de filtration avec le fermenteur, ce module comprenant ici une cellule de filtration à membrane minérale de la figure 4, précédemment décrite.

On commence la fermentation selon le procédé Batch décrit dans l'exemple 13. Après 30 heures de fermentation, on procède à l'alimentation continue avec le milieu nutritif de composition D du tableau II à 60 grammes/litre de glucose et ne contenant pas d'isoleucine qui constitue une substance bloquant la croissance de la souche de Corynebacterium glutamicum précitée productrice de valine.

Les débits d'alimentation et soutirage sont fixés comme dans l'exemple 14 afin de satisfaire un taux de dilution de 0,05 $h^{-1}$.

En même temps, on active la filtration (ou l'ultra-filtration) dans la cellule de filtration (22).

Après 6 heures, la concentration en isoleucine est pratiquement épuisée de sorte que la croissance de la souche est sensiblement ou essentiellement bloquée. Cependant, les cellules de souche continuent à excréter de la valine dans le milieu , excrétion qui est ainsi favorisée.

Un régime stationnaire s'établit et est maintenu pendant 34 heures, caractérisé par une biomasse de 9,9 grammes/litre et une teneur en valine de 15 grammes/litre.

La durée totale de la fermentation est de 70 heures.

Le rendement total en valine rapporté au sucre consommé est de 23 % et la productivité en valine rapportée au litre de fermenteur utile par heure est de 0,63 gramme/litre.

On observe ainsi d'une manière tout à fait inattendue pour un homme du métier, une augmentation radicale en rendement total en valine rapporté au sucre consommé avec une excellente productivité.

## TABLEAU I.

### COMPOSITION DES MILIEUX

| | Milieu de fermentation (A) | Milieu de culture (B) |
|---|---|---|
| Mélasse de betteraves (sucres) | 80 g/l | 40 g/l |
| $H_3PO_4$ | 2 g/l | 2 g/l |
| $Mg\ SO_4,\ 7H_2O$ | 1 g/l | 0,5 g/l |
| $(NH_4)_2\ SO_4$ | 1 g/l | 1 g/l |
| pH ajusté après stérilisation | 7,3 | 7,8 |
| biotine | x $\mu$ g/l | 50 $\mu$ g/l |

EP 0 139 592 B1

TABLEAU II.

| | Milieu C (g/l) | Milieu D (g/l) |
|---|---|---|
| Glucose | 90 | 60 |
| Acétate d'ammonium | 30 | 30 |
| Extrait de levure | 10 | 10 |
| Sels minéraux : | | |
| K2HPO4 | 0,5 | 0,5 |
| KH2PO4 | 0,5 | 0,5 |
| MgSO4-7H2O | 0,25 | 0,25 |
| MnSO4 | 0,01 | 0,01 |
| FeSO4 | 0,01 | 0,01 |
| Facteurs de croissance : | | |
| Biotine | 30 µg/l | 30 µg/l |
| Thiamine | 100 µg/l | 100 µg/l |
| Isoleucine | 0,25 g/l | 0 g/l |
| Eau qsp | 1 litre | 1 litre |

pH ajusté à 7,5 avec de l'ammoniaque

L'invention n'est naturellement pas limitée aux modes de réalisation décrits et représentés qui ne sont donnés qu'à titre d'exemples. L'invention comprend donc tous les moyens constituant des équivalents techniques des moyens décrits ainsi que leurs diverses combinaisons.

La souche productrice d'acide glutamique est de préférence une souche de Corynebacterium et mieux une souche de Corynebacterium melassecola ou de Corynebacterium glutamicum. Le milieu de fermentation ou de culture est également de préférence à base de mélasse de betterave tandis que le substrat nutritif est avantageusement constitué essentiellement par de la mélasse de betterave.

D'autre part, il est avantageux que la concentration en sucre du substrat nutritif utilisé lors de l'étape d'ultra-filtration soit inférieure à celle du substrat nutritif lors de la première étape de fermentation selon le procédé "fed-batch".

**Revendications**

1. Procédé de production d'amino-acide par fermentation comprenant dans une zone de fermentation et dans des conditions de fermentation appropriée, la fermentation d'une souche productrice d'amino-acide sur un milieu de fermentation approprié, de préférence avec ajout d'un substrat nutritif en cours de fermentation selon le procédé "fed-batch", caractérisé en ce que , lorsque l'on atteint une concentration prédéterminée en amino-acide, on procède à une filtration appropriée du milieu de fermentation en obtenant d'une part un rétentat contenant les cellules de la souche et d'autre part un filtrat contenant l'amino-acide ; on recycle au moins en partie le rétentat dans la zone de fermentation et on recueille le filtrat pour de préférence en séparer ensuite l'amino-acide ; et, lors de cette étape de filtration, on bloque sensiblement la croissance de la souche.

2. Procédé selon la revendication 1, caractérisé en ce que l'on réalise la filtration en continu et on introduit du substrat nutritif dans la zone de fermentation en quantité suffisante pour compenser la quantité de substrat soutirée et non réintroduite dans la zone de fermentation.

3. Procédé selon la revendication 1 ou 2, caractérisé en ce que, pour certaines souches, en particulier pour les souches productrices d'acide glutamique, on bloque la croissance de la souche par ajout, par intermittence ou en continu, dans la zone de fermentation, d'au moins un agent bloquant sensiblement la croissance de la souche.

4. Procédé selon la revendication 3, caractérisé en ce que l'agent précité bloquant la croissance de la souche est un dérivé d'acide gras de préférence choisi parmi un ester d'acide gras polyoxyéthyléné, avantageusement du polyoxythylène monopalmitate ou monostéarate et/ou un sel d'acylamine avantageusement l'acétate de laurylamine ou de myristylamine.

5. Procédé selon la revendication 1 ou 2, caractérisé en ce que pour certaines souches, en particulier dans le cas d'une souche productrice de valine, on bloque la croissance de la souche par suppression dans le substrat nutritif ajouté en cours de fermentation, d'une substance essentielle à la croissance de la souche.

6. Procédé selon l'une quelconque des revendications 1 à 4, caractérisé en ce que la souche est une source de Corynebacterium, de préférence de Corynebacterium melassecola ou Corynebacterium glutamicum, encore de préférence une souche de Corynebacterium glutamicum déposée à l'Institut Pasteur sous le N° I026 ou au FRI sous le N° FERM -4035.

7. Procédé selon l'une quelconque des revendications 2 à 6, à fermentation aérobie, caractérisé en ce qu'on réalise l'aération en faisant passer le rétentat recyclé dans un hydro-injecteur avant son recyclage dans la zone de fermentation.

8. Procédé selon l'une quelconque des revendications 1 à 7, caractérisé en ce qu'on utilise un milieu de fermentation ou de culture à base de mélasse de betterave tandis que le substrat nutritif est constitué essentiellement par de la mélasse de betterave.

9. Procédé selon l'une quelconque des revendications 1 à 8, caractérisé en ce que la concentration en sucre du substrat nutritif utilisé lors de l'étape de filtration est inférieure à celle du substrat nutritif utilisé lors de la première étape de fermentation selon le procédé "fed-batch".

10. Procédé selon l'une quelconque des revendications 1 à 9, caractérisé en ce qu'il est appliqué à la production d'un amino-acide choisi parmi le groupe consistant en acide glutamique, en valine, acide aspartique, arginine, histidine, isoleucine, leucine, méthionine, ornithine , phénylalanine proline et thréonine et est de préférence appliqué à la production d'acide glutamique ou de valine.

**11.** Procédé selon la revendication 4, caractérisé en ce qu'on introduit deux agents bloquant la croissance de la souche de préférence constitués par le polyoxyéthylène monopalmitate ou monostéarate et l'acétate de laurylamine ou de myristylamine, ces deux agents, lorsqu'ils sont ajoutés par intermittence, sont ajoutés de manière alternée.

**12.** Installation de production d'amino-acide par fermentation, comprenant un fermenteur (1), des moyens d'alimentation (18) en milieu de fermentation ou en substrat nutritif audit fermenteur, des moyens de soutirage (21) du milieu fermenté du fermenteur, reliés par une pompe (23) à des moyens de soutirage (22) eux-mêmes reliés à l'entrée d'une unité de filtration (22) comprenant une sortie de rétentat (28) et une sortie de filtrat (25); la sortie de rétentat (28) étant reliée au fermenteur (1) pour réaliser au moins en partie un recyclage du rétentat; l'installation comprenant de plus de préférence des moyens d'agitation (2; 36) ainsi que des moyens d'aération (15; 36), caractérisée en ce que l'installation est munie de moyens (50) d'introduction dans le fermenteur (1) d'au moins un agent bloquant la croissance de la souche, tels qu'un tube plongeant.

**13.** Installation selon la revendication 12, caractérisée en ce que l'unité de filtration (22) comprend au moins un module de filtration tangentielle.

**14.** Installation selon la revendication 12, caractérisée en ce que l'unité de filtration (22) comprend au moins un module de filtration de préférence à membrane de préférence minérale.

**15.** Installation selon l'une quelconque des revendications 12 à 14, caractérisée en ce que les moyens d'agitation et/ou d'aération comprennent un hydro-injecteur (36) sur le circuit de recyclage (29) du rétentat (28) au fermenteur (1) dans lequel passe le rétentat recyclé au fermenteur.

**16.** Installation selon l'une quelconque des revendications 12 à 14, caractérisée en ce qu'elle est appliquée à la production d'un amino-acide choisi parmi le groupe consistant en acide glutamique, valine, acide aspartique, arginine, histidine, isoleucine, leucine, méthionine, ornithine, phénylalanine, proline et thréonine, et de préférence est appliquée à la production d'acide glutamique ou de valine.

**Claims**

**1.** Method of producing amino-acids by fermentation comprising a fermentation zone and under suitable fermentation conditions, the fermentation of a strain producing the amino-acid on a suitable fermentation medium, preferably with an addition of nutrient substrate in the course of fermentation according to the "fed-batch" process, characterized in that when one reaches a predetermined amino-acid concentration, one proceeds with a suitable filtering of the fermentation medium obtaining on the one hand a filtration residue containing the cells of the strain and on the other hand a filtrate containing the amino-acid ; one recycles at least in part the filtration residue into the fermentation zone and one collects the filtrate for preferably thereafter separating the amino-acid therefrom and during this filtration step one substantially inhibits the growth of the strain.

**2.** Method according to claim 1, characterized in that one performs the filtration continuously and one introduces nutrient substrate into the fermentation zone in a sufficient amount to compensate for the amount of substrate drawn off and not reintroduced into the fermentation zone.

**3.** Method according to claim 1 or 2, characterized in that with certain strains in particular with the glutamic-acid producing strains, one inhibits the growth of the strain by an intermittent or continuous addition into the fermentation zone of at least one agent substantially inhibiting the growth of the strain.

**4.** Method according to claim 3, characterized in that the aforesaid agent inhibiting the growth of the strain is a fatty acid derivative preferably selected among a polyoxyethylene fatty acid, advantageously polyoxyethylene monopalmitate or monostearate, and/or an acylamine salt advantageously laurylamine or myristylamine acetate.

**5.** Method according to claim 1 or 2, characterized in that for certain strains in particular in the case of a valine-producing strain, one inhibits the growth of the strain by suppression in the nutrient substrate added in the course of fermentation, of a substance essential to the growth of the strain.

6. Method according to any one of claims 1 to 4, characterized in that the strain is a strain of Corynebacterium, preferably of Corynebacterium melassecola or Corynebacterium glutamicum, yet preferably a strain of Corynebacterium glutamicum deposited with the Institut Pasteur under N° 1026 or with the FRI under N° FERM-4035.

7. Method according to any one of claims 2 to 6 with an aerobic fermentation, characterized in that one carries out the ventilation by passing the recycled filtration residue into a hydro-injector before its recycling into the fermentation zone.

8. Method according to any one of claims 1 to 7, characterized in that one uses a fermentation or culture medium based on beet molasses whereas the nutrient substrate is constituted essentially by beet molasses.

9. Method according to any one of claims 1 to 8, characterized in that the sugar concentration of the nutrient substrate used in the filtration stage is lower than that of the nutrient substrate used in the first fermentation stage according to the "fed-batch" process.

10. Method according to any one of claims 1 to 9, characterized in that it is applied to the production of an amino-acid selected among the group consisting of glutamic acid, valine, aspartic acid, arginine, histidine, iscleucine, leucine, methionine, ornithine, phenylalanine, proline and threonine and is preferably applied to the production of glutamic acid or of valine.

11. Method according to claim 4, characterized in that one introduces two agents inhibiting the growth of the strain and preferably constituted by the polyoxyethylene monopalmitate or monostearate and by laurylamine or myristylamine acetate, which two agents, when they are added intermittently, are added alternately.

12. Equipment for the production of amino-acid by fermentation, comprising a fermenter (1), means (18) for feeding a fermentation medium or a nutrient substrate to the said fermenter, means (21) for drawing off fermented medium from the fermenter, connected by a pump (23) to draw-off means (22) themselves connected to the inlet of a filtration unit (22) comprising an outlet (28) for the filtration residue and an outlet (25) for the filtrate; the outlet (28) for the filtration residue being connected to the fermenter (1) for carrying out at least in part a recycling of the filtration residue; the equipment further preferably comprising stirring means (2;36) as well as ventilation means (15;36), characterized in that the equipment is provided with means (50) for the introduction into the fermenter (1) of at least one agent inhibiting the growth of the strain such as a plunger tube.

13. Equipment according to claim 12, characterized in that the filtration unit (22) comprises at least one tangential filtering module.

14. Equipment according to claim 12, characterized in that the filtration unit (22) comprises at least one filtration module preferably having a preferably mineral membrane.

15. Equipment according to any one of claims 12 to 14, characterized in that the stirring and/or ventilation means comprise a hydro-injector (36) on the circuit (29) for the recycling of the filtration residue (28) to the fermenter (1), in which passes the filtration residue recycled to the fermenter.

16. Equipment according to any one of claims 12 to 14, characterized in that it is applied to the production of an amino-acid selected among the group consisting of glutamic acid, valine, aspartic acid, arginine, histidine, iscleucine, leucine, methionine, ornithine, phenylalanine, proline and threonine and preferably is applied to the production of glutamic acid or of valine.

**Patentansprüche**

1. Verfahren zur Erzeugung von Aminosäure durch Gärung mit in einem Gärungsbereich und unter geeigneten Gärungsbedingungen, die Gärung eines Aminosäure erzeugenden Stammes auf einem geeigneten Gärmedium, vorzugsweise mit Zusatz eines Nährsubstrats im Laufe der Gärung gemäss dem "Fed-Batch"- Verfahren, dadurch gekennzeichnet, dass, wenn man eine vorbestimmte Konzentra-

EP 0 139 592 B1

tion an Aminosäure erreicht, man eine geeignete Filtrierung des Gärmediums duchführt, wobei einerseits ein die Zellen des Stammes enthaltender Stoff und andererseits ein die Aminosäure enthaltendes Filtrat erhalten wird; man wenigstens ein Teil des zurückgehaltenen Stoffes in den Gärbereich zurückführt und man das Filtrat einsammelt, vorzugsweise um nachher die Aminosäure davon abzutrennen; und man während diesem Filtrierungsvorgang das Wachsen des Stammes im wesentlichen hemmt.

2. Verfahren nach Anspruch 1, dadurch gekennzeichnet, dass man die Filtrierung fortlaufend durchführt und man mehr Substrat in den Gärbereich in ausreichender Menge einführt, um die abgezogene und in den Gärbereich nicht wiedereingeführte Substratmenge auszugleichen.

3. Verfahren nach Anspruch 1 oder 2, dadurch gekennzeichnet, dass bei gewissen Stämmen, insbesondere bei den Glütaminsäure erzeugenden Stämmen, man das Wachsen des Stammes durch intermittierenden oder fortlaufenden Zusatz wenigstens eines das Wachsen des Stammes im wesentlichen hemmenden Mittels hemmt.

4. Verfahren nach Anspruch 3, dadurch gekennzeichnet, dass das vorgenannte, das Wachsen des Stammes hemmende Mittel ein vorzugsweise unter einem Polyoxyethylenfettsäureester, vorteilhaft Polyoxyethylenmonopalmitat oder-monostearat und/oder einem Acylaminsalz, vorteilhaft Laurylaminacetat oder Myristylaminacetat gewähltes Fettsäurederivat ist.

5. Verfahren nach Anspruch 1 oder 2, dadurch gekennzeichnet, dass, bei gewissen Stämmen, insbesondere in dem Fall eines Valin erzeugenden Stammes, man das Wachsen des Stammes durch Weglassen in dem im Laufe der Gärung zugesetzten Nährsubstrat, einer für das Wachsen des Stammes wesentlichen Substanz hemmt.

6. Verfahren nach irgendeinem der Ansprüche 1 bis 4, dadurch gekennzeichnet, dass der Stamm ein Corynebacterium-Stamm, vorzugsweise Corynebacterium-Melassecola- oder Corynebacterium-Glutamicum- oder noch vorzugsweise ein beim Institut Pasteur unter Nr 1026 oder beim FRI unter Nr FERM -4035 hinterlegter Corynebacterium-Glutamicum- Stamm ist.

7. Verfahren nach irgendeinem der Ansprüche 2 bis 6, mit aeroben Gärung, dadurch gekennzeichnet, dass man die Belüftung durch Einführung des rückgeführten zurückgehaltenen Stoffes in einen Hydro-Injektor vor seiner Rückführung in den Gärbereich durchführt.

8. Verfahren nach irgendeinem der Ansprüche 1 bis 7, dadurch gekennzeichnet, dass man ein Zuckerrübenmelassebasisches Gär-bzw. Zuchtmedium verwendet, während das Nährsubstrat im wesentlichen durch Zuckerrübenmelasse gebildet wird.

9. Verfahren nach irgendeinem der Ansprüche 1 bis 8, dadurch gekennzeichnet, dass die Zuckerkonzentration des verwendeten Nährsubstrates während der Filtrierungstufe niedriger als diejenige des während der ersten Gärstufe gemäss dem "Fed-Batch"-Verfahren verwendeten Nährsubstrates ist.

10. Verfahren nach irgendeinem der Ansprüche 1 bis 9, dadurch gekennzeichnet, dass es an die Erzeugung einer in der aus Glutaminsäure, Valin, Asparaginsäure, Arginin, Histidin, Isoleucin, Leucin, Methionin, Ornithin, Phenylalanin, Prolin, Threonin bestehenden Gruppe gewählten Aminosäure angewandt wird und vorzugsweise an die Erzeugung von Glutamsäure oder Valin angewandt wird.

11. Verfahren nach Anspruch 4, dadurch gekennzeichnet, dass man zwei vorzugsweise aus Polyoxyethylenemonopalmitat oder-monostearat und Laurylamin-oder Myristilaminacetat gebildeten, das Wachsen des Stammes hemmende Mittel einführt, wobei diese beiden Mittel, wenn sie intermittierend zugesetzt werden, in wechselnder Weise zugesetzt werden.

12. Anlage zur Erzeugung von Aminosäure durch Gärung, mit einem Gärer (1), Mitteln (18) zur Zuführung von Gärmedium oder Nährsubstrat zu dem besagten Gärer, Mitteln (21) zum Abziehen des gegärten Mediums aus dem Gärer, welche durch eine Pumpe (23) mit Abziehmitteln (22) verbunden sind, welche selbst mit dem Eintritt einer, einen Austritt (28) für zurückgehaltenen Stoff und einen Filtrataustritt (25) aufweisenden Filtriereinheit (22) verbunden sind; wobei der Austritt (28) für zurückgehaltenen Stoff mit

14

dem Gärer (1) verbunden ist, um wenigstens teilweise eine Rükführung des zurückgehaltenen Stoffes durchzuführen; wobei die Anlage ausserdem vorzugsweise Rührmittel (2;36) sowie Belüftungsmittel (15,36) aufweist, dadurch gekennzeichnet, dass die Anlage mit Mitteln (50) wie einem Tauchrohr zum Einführen wenigstens eines das Wachsen des Stammes hemmenden Mittels in den Gärer (1) versehen ist.

13. Anlage nach Anspruch 12, dadurch gekennzeichnet, dass die Filtrierungseinheit (22) wenigstens einen Modul zur tangentialen Filtrierung umfasst.

14. Anlage nach Anspruch 12, dadurch gekennzeichnet, dass die Filtrierungseinheit (22) wenigstens einen Filtrierungsmodul vorzugsweise mit einer vorzugsweise mineralischen Membran aufweist.

15. Anlage nach irgendeinem der Ansprüche 12 bis 14, dadurch gekennzeichnet, dass die Rühr-und/oder Belüftungsmittel einen Hydro-Injektor (36) in dem Kreislauf (29) zur Rückführung des zurückgehaltenen Stoffes (28) zu dem Gärer (21) in welchem der zu dem Gärer rückgeführte zurückgehaltene Stoff fliesst, aufweisen.

16. Anlage nach irgendeinem der Ansprüche 12 bis 14, dadurch gekennzeichnet, dass sie an die Erzeugung einer in der aus Glutaminsäure, Valin, Asparaginsäure, Arginin, Histidin, Isoleucin, Leucin, Methionin, Ornithin, Phenylalanin, Prolin und Threonin gebildeten Gruppe gewählten Aminosäure angewandt wird und vorzugsweise an die Erzeugung von Glutaminsäure oder Valin angewandt wird.

Fig. 1

Fig. 2

Fig. 3

Fig. 4

Fig. 5

Fig. 6